# EUROPEAN PATENT APPLICATION

(11) **EP 1 806 574 A1**
(43) Date of publication of application: **11.07.2007**
(21) Application number: 06447001.6
(22) Date of filing: 05.01.2006
(51) Int. Cl.: G01N 21/55, G01N 33/543

(54) **Surface modification of optical elements for the spectroscopic detection of molecules and organic components**

(71) Applicant: Université Catholique De Louvain, 1348 Louvain-La-Neuve (BE); Université Libre de Bruxelles, 1050 Bruxelles (BE); Université de Mons-Hainaut, 7000 Mons (BE)
(72) Inventor: Conti, Joséphine, 7390 Quaregnon (BE); De Coninck, Joël Joseph Florent, 7022 Mesvin (BE); Devouge, Sabrina, 6880 Bertrix (BE); Salvagnini, Claudio, 1300 Limal (BE); Marchand-Brynaert, J. A-M.G., Univ.Catholique ..., 1348 Louvain-la-Neuve (BE); Homblé, Fabrice Roland, 1440 Braine-le-Château (BE); Goormaghtigh, Erik Robert Marcel Charles, 7850 Enghien (BE); Barbar, Sajid, 1081 Bruxelles (BE)
(74) Representative: Brants, Johan P.E.

(57) **Abstract**

The invention relates to a device suitable for the investigation of ligand-receptor interactions, in particular for the investigation of an analyte target interaction such as biological and chemical molecules and organic components and their interaction with surfaces consisting of an attenuated total internal reflection element, transparent in the infra-red and of which at least one surface is reduced and covalently grafted with an optionally substituted alkene able to immobilize the receptor.

## Description

The invention relates to devices suitable for the investigation of ligand-receptor interactions, in particular for the investigation of biological or chemical molecules and organic components and their interaction with suitably modified surfaces. More in particular the invention concerns methods of surface modification and covalent grafting of ATR (Attenuated Total Internal Reflection) elements and their use in FTIR (Fourier Transform Infra Red) devices for the detection, characterization, and dosage of biological molecules and organic compounds.

### Background of Art

Biosensors (for a detailed review, see: Leech D, Chem. Soc. Rev., 1994, 23, 205-213) are devices based on the specific recognition of an analyte of interest by a target such as a biological component, for example a receptor, an antibody, an enzyme, a membrane, a cell or cell containing media, a molecule and the subsequent transformation of this interaction into an electrical, optical, or other signal.

Different surface sensitive techniques can be applied to detect ligand-receptor interactions, depending on the nature of the sensor supports. Fourier transform infra-red (FTIR) spectroscopy is an extremely powerful analytical technique, particularly well adapted to the characterization of organic molecules and biological systems. Quantitative structural and conformational information can be recorded. The method has been applied to the investigation of mono- and multilayers of bio-organic samples displayed on the optical elements, by using the attenuated total internal reflection (ATR) configuration (Scheuing DR, Fourier Transform Infra-Red Spectroscopy in Colloid and Interface Science, ACS Symposium Series n° 447, Am. Chem. Soc., Washington, 1991; Mirabella Jr FM (editor), Internal Reflection Spectroscopy, Theory and Applications, Marcel Dekker, New York, 1993). Interestingly, the ATR configuration allows the study of analytes such as biological components and molecules or proteins, for instance on surfaces in contact with water-containing media. The examination of protein adsorption on biomaterial surfaces is one of the relevant applications (Chittur KK, Biomaterials, 1998, 19, 357). Several techniques are available to immobilize molecules of interest on ATR crystals, such as the coating with thin layers of synthetic polymers, such as polyurethanes, polystyrene, polyethylene by spin-casting (Lenk TJ, Ratner BD, Chittur KK, Gendreau RM, Langmuir, 1991, 7, 1755; Lenk TJ, Ratner BD, Chittur KK, Gendreau RM, J. Biomed. Mater. Res., 1989, 23, 549), and the coating with bioceramic, such as hydroxyapatite by ion bombardment (Ong JL, Chittur KK, Lucas LC, J. Biomed. Mater. Res., 1994, 28, 1337). The main disadvantage of the use of an intermediate film of metal, polymer, or ceramic on the surface of the ATR element is the formation of a barrier for light transmission, which results in a drastic restriction of the possibilities to detect ligand-receptor interactions taking place at the ATR surface.

WO 02/056018 describes methods of chemical surface oxidation and covalent grafting of ATR with silanes and derivatives thereof, and devices obtained therewith. However, there is still a need for an improved method of surface modification of ATR elements which provides stable surface grafted ATR elements.

### Summary of the invention

In a first aspect, the present invention provides a device suitable for the investigation of ligand-receptor interactions, in particular for the investigation of an analyte target interaction such as biological and chemical molecules and organic components and their interaction with surfaces, consisting of an attenuated total internal reflection element, transparent in the infra-red and of which at least one surface is reduced and covalently grafted with an alkene able to immobilize the receptor, wherein said alkene is optionally substituted by one or more substituent selected from alkyl, haloalkyl, halo, alkenyl, cyano, epoxy, thio, amino, hydroxyl, isocyano, isothiocyano, carboxy, polyalkoxy, alkyarylsulphoxypolyalkoxy, heteroaryloxycarbonylakylpolyalkoxy. Preferably, said optionally substituted alkene is of Formula 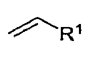 wherein R¹ is selected from CₙH₂ₙ₊₁, CₙF₂ₙ₊₁, -(CH2)ₘ-(O-CH₂-CH₂)ₚ-OR², wherein R² is selected from C₁₋₃alkyl, alkyarylsulphoxide, heteroaryloxycarbonylakyl, 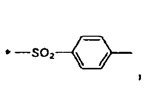 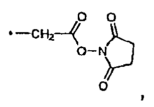 or mixture thereof,
wherein n is an integer from 3 to 50, wherein m is an integer from 0 to 20, and wherein p is an integer from 3 to 20. According to the invention said optionally substituted alkene can be covalently coupled with a multifunctional spacer-arm of the general formula Z¹-(CH₂)_{q}-Z² wherein q is 2 to 12; or Z¹-CH₂-(O-CH₂-CH₂)_{q}-OCH₂-Z² wherein q' is 0 to 5; wherein Z¹, Z² are each independently chosen from N₃-aryl-; diazirinyl; -CO₂H and N-hydroxysuccinimidyl ester thereof; -CH₂-NH₂ and N-maleimidyl derivative thereof; -CH₂OH and tosylates thereof; -CH₂-SH and dithiane derivatives thereof; -CH₂-N=C=O; -CH₂N=C=S; 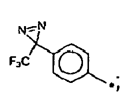 or 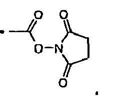 In another aspect, the present invention also relates to a method for the construction of a device according to the invention comprising the steps of: - reduction of at least one surface of an attenuated total internal reflection element, - surface grafting with an alkene of the reduced surface obtained in the previous step, and - coupling a receptor via covalent fixation on the grafted element.

The first step consists of reducing the ATR element surface. According to the present invention, ATR elements can be made of silicon or germanium crystals. In a preferred embodiment, said ATR element is made of germanium.

The second step consists of grafting of the reduced surface with an alkene through covalent coupling. Reduction of the ATR surface allows an easier and direct grafting of the alkene on this surface. This results in a more accurate and efficient measuring of ligand-receptor interactions via infra-red spectroscopy in the nanomolar range. The present device is prepared by grafting one or more alkenes on the reduced surface of the ATR. The present devices provide several advantages compared to the prior art: Alkenes are readily available compounds, which when grafted to the reduced ATR surface are very stable and not prone to hydrolysis. The device displays enhanced stability compared to prior art. ln addition, upon use of the device in FTIR, there is no more masking of part of the spectrum by Si-O or Ge-O band when compared to prior art device. This allows reduction of the noise of the surface modified ATR element.

In a further aspect, the present invention also relates to a method for studying ligand-receptor interactions, in particular biological molecules or organic components or their interactions or complexations or reactions with biological molecules or organic components or water-soluble molecules at or in the grafted organic molecule, using a device according to the invention.

The present invention is directed to a device suitable for the investigation of ligand-receptor interactions, in particular for the investigation of an analyte target interaction such as biological and chemical molecules and organic components and their interaction with surfaces, consisting of an attenuated total internal reflection element, transparent in the infra-red and of which at least one surface is reduced and covalent!y grafted with an optionally substituted alkene able to immobilize the receptor.

The devices and methods of the present invention are relatively easy to use, and require fewer procedural steps and less complex assay technique, by comparison with prior art assays, and also provide the additional advantage of rapid quantitative, semi-quantitative or qualitative results for testing of samples. The devices are additionally adapted for advantageous use as controls, e.g., to assess the accuracy and reliability of such assays. Moreover, during manufacture, devices of the invention can be relatively easily made. Assays utilizing such devices of the invention have also been found to be highly sensitive to various levels of analytes.

The foregoing advantages, as well as other advantages, will be apparent from the detailed description of the invention as set forth herein, the drawings and the examples illustrating it.

### Description of the figures

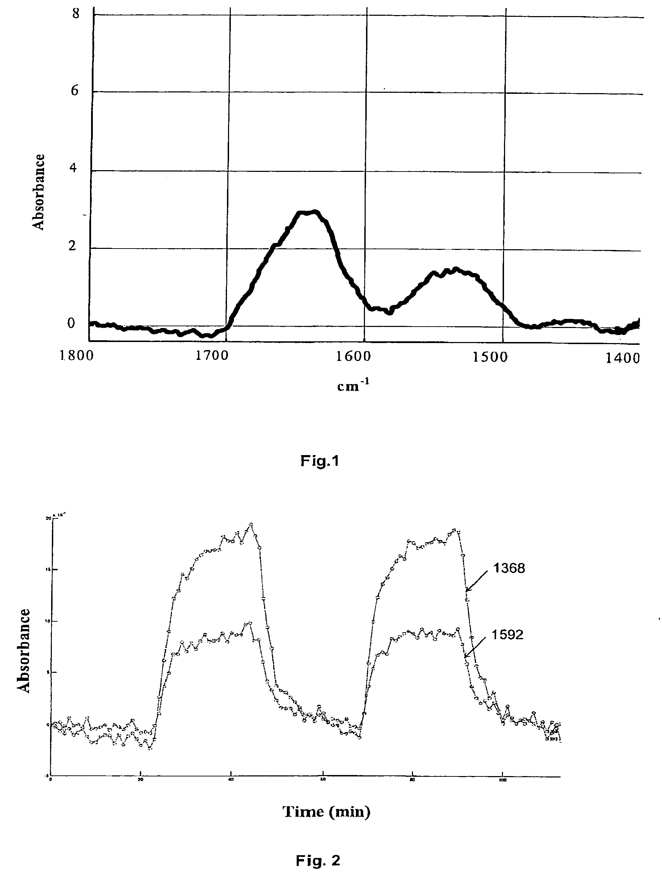

Figure 1 shows an FTIR-ATR spectrum of a receptor covalently attached on the grafted germanium internal reflection element. The spectrum (256 scans, resolution 2 cm⁻¹) was recorded by attenuated total reflection on a 50x20x2 mm trapezoidal germanium crystal. Water contribution has been subtracted.
Figure 2: shows an FTIR-ATR spectrum recorded as a function of time. The evolution of the infrared spectrum was monitored at 1550 cm⁻¹ and 1395 cm⁻¹ upon injection of the sulfadimethoxine solution in the ATR flow cell. The spectra (256 scans, resolution 2 cm⁻¹) were recorded by attenuated total reflection. Water contribution has been subtracted. The rise of the intensity corresponds to the introduction of the drug solution in the chamber while the decays correspond to the washing with the buffer.

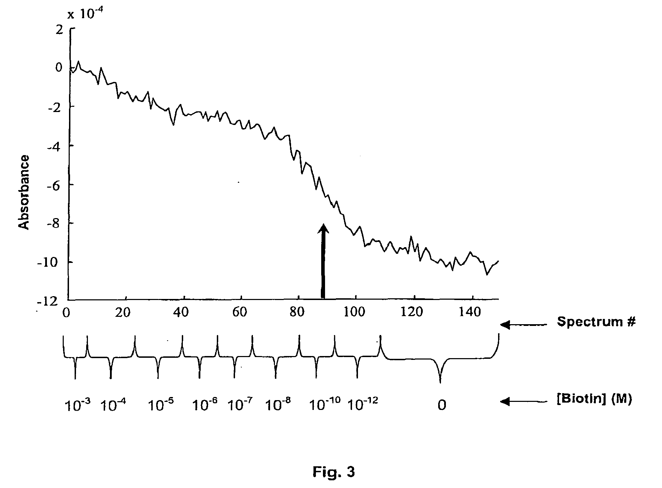

Figure 3 shows an FTIR-ATR spectrum recorded as a function of biotine concentration. The evolution of the infrared spectrum was monitored at 1627 cm⁻¹ as a function of the biotin concentration in the ATR flow cell. The spectra (256 scans, resolution 2 cm⁻¹) were recorded by attenuated total reflection. Water contribution has been subtracted.

### Detailed description of the Invention

The invention provides a device suitable for the investigation of ligand-receptor interactions, in particular for the investigation of an analyte target interaction such as biological and chemical molecules and organic components and their interaction with surfaces, consisting of an attenuated total internal reflection element, transparent in the infra-red and of which at least one surface is reduced and covalently grafted with an alkene optionally substituted by one or more substituents, preferably 1, 2 or 3 substitutents selected from alkyl, haloalkyl, halo, alkenyl, cyano, epoxy, thio, amino, hydroxyl, isocyano, isothiocyano, carboxy, polyalkoxy, alkyarylsulphoxypolyalkoxy, heteroaryloxycarbonylakylpolyalkoxy.

According to the present invention, ATR elements can be made from a material chosen from the group consisting of germanium, silicon, ZnSe, ZnS, AM-TIR (an amorphous glass of germanium, selenium and arsenic). In a preferred embodiment, said ATR element is made of germanium. Preferably, the attenuated total internal reflection element is a crystal, preferably having a trapezoidal, hemi-cylindrical, fiber or rod shaped geometry, or any polyhedral form usable in optical application.

The present invention also encompasses a method for the preparation of a device according to the invention comprising the steps of: - reducing of at least one surface of an attenuated total internal reflection element, - surface grafting with an alkene of the reduced surface obtained in the previous step, and - coupling a receptor via covalent fixation on the alkene.

When describing the present invention, the terms used are to be construed in accordance with the following definitions, unless a context dictates otherwise:

The term "alkene" refers to a straight or branched alkane chain containing at least one unsaturation in the form of a single carbon to carbon double bond and having 3 to 52 carbon atoms, for example 3 to 40 carbon atoms.

The term "alkyl" by itself or as part of another substituent, refers to a straight or branched saturated hydrocarbon group joined by single carbon-carbon bonds having 1 to 10 carbon atoms, for example 1 to 8 carbon atoms, for example 1 to 6 carbon atoms, preferably 1 to 4 carbon atoms. When a subscript is used herein following a carbon atom, the subscript refers to the number of carbon atoms that the named group may contain. Thus, for example, C₁₋₄alkyl means an alkyl of one to four carbon atoms. Examples of alkyl groups are methyl, ethyl, propyl, isopropyl, butyl, isobutyl, sec-butyl, *tert*-butyl, 2-methylbutyl, pentyl iso-amyl and its isomers, hexyl and its isomers, heptyl and its isomers and octyl and its isomers. Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene.

The term "alkenyl" by itself or as part of another substituent, refers to a straight or branched alkyl chain containing at least one unsaturation in the form of a single carbon to carbon double bond and having 2 to 10 carbon atoms, for example 2 to 8 carbon atoms, preferably 2 to 6 carbon atoms, more preferably 2 to 4 carbon atoms. Examples of alkenyl groups are ethenyl, 2-propenyl, 2-butenyl, 3-butenyl, 2-pentenyl and its isomers, 2-hexenyl and its isomers, 2-heptenyl and its isomers, 2-octenyl and its isomers, 2,4-pentadienyl and the like.

The term "aryl" as used herein by itself or as part of another group refers but is not limited to 5 to 14 carbon-atom homocyclic (i.e., hydrocarbon) monocyclic, bicyclic or tricyclic aromatic rings or ring systems containing 1 to 4 rings which are fused together or linked covalently, typically containing 5 to 8 atoms; at least one of which is aromatic. Where aryl groups as defined are divalent, i.e., with two single bonds for attachment to two other groups, they are termed "arylene" groups.

The terms "heterocyclyl" as used herein by itself or as part of another group refer to an optionally substituted non-aromatic, fully saturated or partially unsaturated cyclic groups (for example, 3 to 13 member monocyclic, 7 to 17 member bicyclic, or 10 to 20 member tricyclic ring systems, or containing a total of 3 to 10 ring atoms) which have at least one heteroatom in at least one carbon atom-containing ring, optionally substituted with 1, 2 or 3 substituents selected from oxy, alkyl, halo, haloalkyl, carboxy, amino or sulphonyl . Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. Exemplary heterocyclic groups include maleimidyl, succinimidyl, piperidinyl, imidazolinyl, imidazolidinyl, isoxazolinyl, oxazolidinyl, isoxazolidinyl, thiazolidinyl, isothiazolidinyl, piperidyl, succinimidyl, 3H-indolyl, indolinyl, isoindolinyl, chromenyl, isochromanyl, xanthenyl, 2H-pyrrolyl, 1-, 2-, 3-pyrrolinyl.

The term "amino" refers to the group -NH₂.

The term "halo" or "halogen" as a group or part of a group is generic for fluoro, chloro, bromo or iodo.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoromethyl, 1,1,1-trifluoroethyl and the like.

The term "epoxy" as used herein refers to cyclic ether with only three ring atoms.

The term "thio" as used herein refers to the -SH group.

The term "isocyano" as used herein refers to the group of formula -N=C=O.

The term "isothiocyano" as used herein refers to a group of formula -N=C=S.

The term "polyalkoxy" as used herein refers to a group of formula -(O-R³)ₚ-OR⁴, wherein p is an integer from 1 to 8, and R³ is alkylene and R⁴ is alkyl.

The term "alkyarylsulphoxypolyalkoxy" as used herein refers to a group of formula -(O-R³)ₚ-OSO₂-R⁵-R⁶, wherein p is an integer from 1 to 8, and R³ is alkylene, wherein R⁵ is arylene and R⁶ is alkyl.

The term "heteroaryloxycarbonylakylpolyalkoxy", as used herein refers to a group of formula -(O-R³)ₚ-CO-O-R⁷, wherein p is an integer from 1 to 8, and R³ is alkylene and R⁷ is heterocyclyl, preferably maleimidyl or succenimydyl.

As used in the specification and the appended claims, the singular forms "a", "an," and "the" include plural referents unless the context clearly dictates otherwise. By way of example, "an alkene" means one alkene or more than one alkene.

Asterisks (*) are used herein to indicate the point at which a mono-, bi- or trivalent radical depicted is connected to the structure to which it relates and of which the radical forms part.

ln general, the present invention provides a method to reduce the surface of an optical element, transparent in the infra-red, particularly a silicon or germanium ATR device, typically a crystal, an optical fiber or a rod made of such materials, and to further covalently fix an optionally functionalized alkene able to immobilize biological components and molecules, including biological and non-biological receptors, proteins, antibodies, membranes, and the like. This purposely modified ATR element provides a method to study ligand-receptor interactions occurring at the solvent ATR element interface, particularly, at the water-containing media - ATR element interface, by using attenuated total internal reflection (ATR) infra-red (IR) spectroscopy, preferably Fourier transform infra-red spectroscopy (FTIR). The technique is based on the reduction of the ATR element followed by the covalent grafting of alkene optionally presenting a functional group at a terminus for the covalent coupling of biological receptors, either directly, or *via* multifunctional spacer-arms.

The first step consists of reducing the ATR element surface. The reduction step can be performed chemically or electrochemically or chemically using a reducing agent. In a preferred embodiment, the surface is first stripped (etched) with an oxidizing agent and then reduced with a reducing agent.

Suitable oxidizing agents for use in the stripping step include but are not limited to: H₂O₂. The stripping step may be performed using from 10 to 50% by weight of oxidizing agent, preferably from 30 to 35%. The temperature is preferably comprised between -15°C and +150°C, preferably between 15° and 35°C, and the duration of the treatment comprised between a few seconds to several hours. In a particular embodiment, it can be performed at room temperature, for a period of 5 to 200 seconds, preferably of 5 to 100 seconds.

Suitable reducing agents for use in the present invention include but are not limited to HF, and ammonium salts thereof (R₃NH⁺F⁻). The reduction step may be performed using from 5 to 30% by weight of reducing agent, preferably from 7 to 20%. It can be performed at room temperature, for a period of 1 min to 1 hour, preferably 10 to 30 min.

The second step consists of grafting of the reduced surface with an alkene through covalent coupling. In a particular embodiment of the present invention, the optionally substituted alkene is of Formula 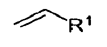 wherein R¹ is selected from CₙH₂₊₁, CₙF₂ₙ₊₁, -(CH₂)ₘ-(O-CH₂-CH₂)ₚ-OR², wherein R² is selected from C₁₋₃alkyl, alkyarylsulphoxide, heteroaryloxycarbonylakyl, 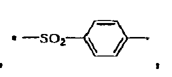 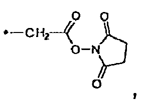 or mixture thereof,
wherein n is an integer from 3 to 50, wherein m is an integer from 0 to 20, and wherein p is an integer from 3 to 20.

The second step can be performed using from 0.5 to 10% by weight of alkene as defined herein, preferably from 1 to 5%. Thermal or photochemical treatments are preferred for this step. The reaction can be performed at 120 to 180 °C for about 1 to 3 hours.

According to the present invention, the present method may further comprise covalently coupling the modified ATR with a multifunctional spacer of the general formula Z¹-(CH₂)_{q}-Z² wherein q is 2 to 12; or Z¹-CH₂-(O-CH₂-CH₂)_{q}-OCH₂-Z² wherein q' is 0 to 5; wherein Z¹, Z² are each independently chosen from N₃-aryl-; diazirinyl; -CO₂H and N-hydroxysuccinimidyl ester thereof; -CH₂-NH₂ and N-maleimidyl derivative thereof; -CH₂OH and tosylates thereof; -CH₂-SH and dithiane derivatives thereof; -CH₂-N=C=O; -CH₂N=C=S; 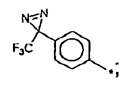 or

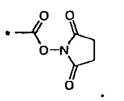

The covalent coupling on the modified ATR element is obtained by contacting a solution of multifunctional molecules as described herein with the modified ATR element.

The same experimental conditions as described for step 2 apply. In the case of Z¹, Z² equal to N₃-aryl or diazirinyl, light activation is applied (hλ between 200 and 600 nm).

The last step comprises immobilizing a receptor on the grafted molecule. The modified ATR element is placed in contact with a water-containing solution of receptor, preferentially proteins, peptides, membranes, and the like such as avidine, albumin, PE-biotine and the like. Biologically active small molecules, such as biotine, are also suitable receptors.

The concentration is preferably in the range 10⁻⁶ mg/ml to 10³ mg/ml, the temperature is comprised between -15°C and 150°C and the interaction time is from a few seconds to several hours (or up to several hours, if accepted).

The activated functions of the surface modified ATR element react as such; it is the case, for instance, for isocyanate, isothiocyanate, ester of N-hydroxysuccinimide, N-maleimide, tosylate. The non-activated functions, such as free acid, amine, alcohol, they can be activated before hand in situ by using the classical methods of peptide synthesis.

For the FTIR-ATR detection and quantification of ligand-receptor interaction, the ATR element comprising the receptor is placed in an FTIR cell and submitted to a flux of potential ligands, preferably in a water-containing solution.

The present invention also encompasses the use of the present device for studying ligand-receptor interactions, in particular biological molecules or organic components or their interactions or complexations or reactions with biological molecules or organic components or water-soluble molecules at or in the grafted alkene.

The present invention also encompasses a method for reducing a surface of an attenuated total internal reflection element, comprising the step of stripping the surface with an oxidant and reducing the stripped surface with a reducing agent. In an embodiment, the reduction of the attenuated total internal reflection element can be performed by the consecutive treatment of a surface thereof with H₂O₂ and then HF. In a particular embodiment, the reduction of a germanium crystal is performed by the consecutive treatment of a surface thereof with H₂O₂ and then HF.

The present invention also encompasses a method for studying ligand-receptor interactions, in particular biological molecules or organic components or their interactions or complexations or reactions with biological molecules or organic components or water-soluble molecules at or in the grafted organic molecule, using a device according to the present invention comprising the steps of - installation of said device in a FTIR cell, - conducting a flux of potential ligands, preferably a water-containing solution, on said device surface, - analysis of the infra-red spectrum obtained after submitting a FTIR beam through said cell, and - reuse of said device surface by application of a solution of free ligand.

In general, the inventive method allows the study of specific ligands interacting with the receptor fixed on the ATR element. The element is placed in a low pressure cell allowing a liquid flux to pass on its surface. A solution of potential ligands to be analyzed is passed through the cell submitted to FTIR beam.

Due to the low penetration depth of the evanescent field, ligands in solution do not significantly contribute to the lR spectrum. On the contrary, ligands fixed on the receptors, close to the surface of the ATR element, increase considerably the local concentration at the interface, and contribute to the lR spectrum. The ATR element is preferably a crystal, more preferably having a trapezoidal, hemi - cylindrical, fiber or rod shaped geometry, or any polyhedral form usable in optical application. These types of crystals are easy to use and allow good detection.

The quantitative analysis of fixed ligands is an object of the invention. The time-resolved study of ligand-receptor interaction is a further object of the invention. The reuse of the ATR element is another object of the invention. Once the assay performed ATR element surface can be reused. The fixed ligand from the previous step can be displaced from the ATR element surface by the application of a solution of free ligand.

### Examples

The devices according to the invention may be prepared according to the following schemes 1, 2 or 3.

Scheme 1 illustrates the preparation of a hydrophobic biosensor according to an embodiment of the present invention. Step (i) comprises the reduction of the crystal (Ge or Si). Said reduction can be performed with HF or electrochemically. Step (ii) comprises the grafting of an aliphatic alkene, also referred as hydrogermylation. This step can comprise a thermal or photochemical activation. Step (iii) comprises the grafting of activated esters in the organic layer by photochemistry. Suitable molecules which can be photoactivated include but are not limited to nitrene precursors A and carbene precursors B.

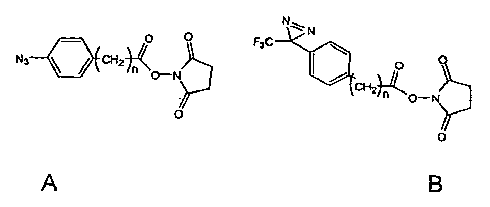

The last step (iv) comprises the covalent binding of the biological receptor.

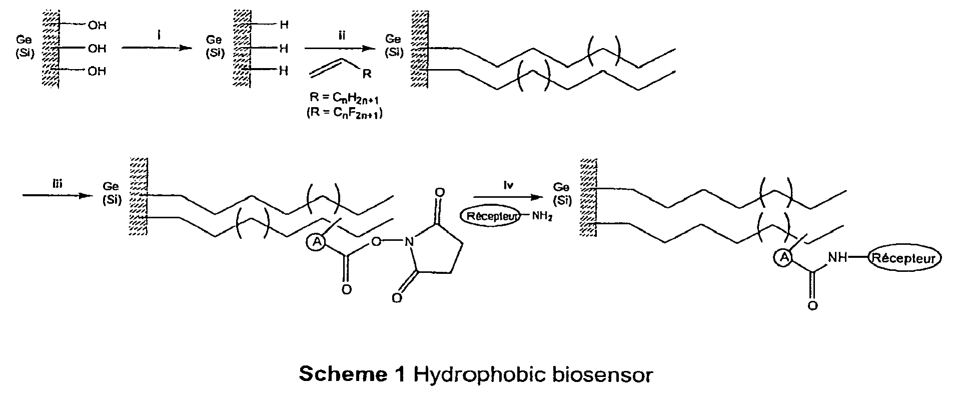

Scheme 2 illustrates part of the preparation process of hydrophilic or mixed biosensor according to embodiments of the present invention, wherein steps (i), (iii) and (iv) (not shown) are identical to steps (i), (iii) and (iv) of Scheme 1, and step (ii) is modified and comprises the grafting of polyalkyloxyalkenes on the reduced Ge or Si surface. Suitable polyalkyloxyalkenes are compounds of formula CH₂=CH-(CH₂)ₘ-(O-CH₂-CH₂)p-OCH_{3.} wherein m is an integer ≤ 2 or ≥ 8 and p is an integer selected from 3, 4, 5, 6, 7, 8. When polyalkyloxyalkenes of formula I with m is ≤ 2 and p is = 3 to 8 is used a hydrophilic biosensor is obtained. When polyalkyloxyalkenes of formula I with m is ≥ 8 and p is = 3 to 8 is used a mixed biosensor is obtained.

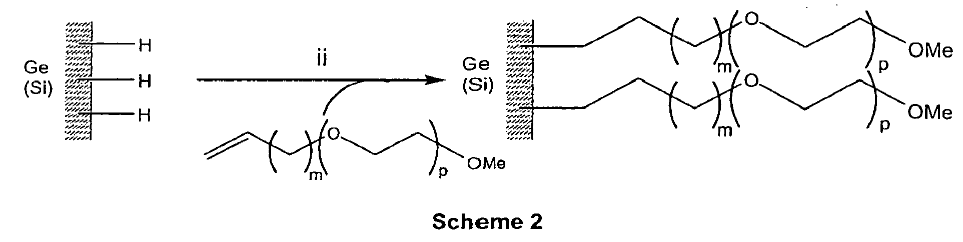

Scheme 3 illustrates part of the preparation process of hydrophilic or mixed biosensor according to embodiments of the present invention, wherein steps (i) and (iv) (not shown) are identical to steps (i) and (iv) of Scheme 1, step (iii) is omitted and step (ii) is modified and comprises the grafting of functional polyalkyloxyalkenes on the reduced Ge or Si surface. Suitable functional polyalkyloxyalkenes are compounds of formula CH₂=CH-(CH₂)ₘ-(O-CH₂-CH₂)ₚ-OR, wherein m is an integer ≤ 2 or ≥ 8, wherein p is an integer selected from 3, 4, 5, 6, 7, 8 and wherein 0 to 100 % of R is selected from 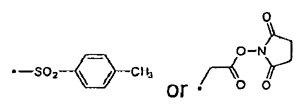 with 100% to 0% of R being -CH₃. When polyalkyloxyalkenes of formula I with m is ≤ 2 and p is = 3 to 8 is used a hydrophilic biosensor is obtained. When polyalkyloxyalkenes of formula I with m is ≥ 8 and p is = 3 to 8 is used a mixed biosensor is obtained.

Step (iii) is not needed anymore as the alkene comprises a functional binding group which allows to proceed with step (iv).

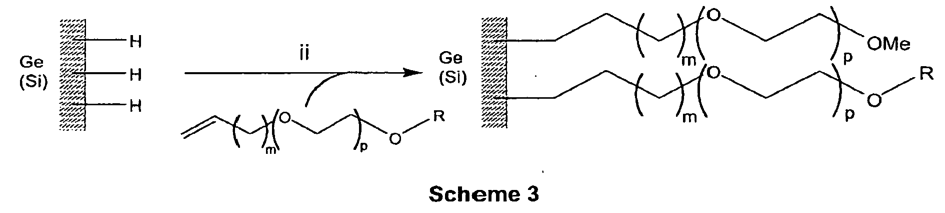

### Example 1: Surface grafting of a perfluorinated alkene on a germanium crystal and its detection by XPS.

### Step 1: surface activation

A germanium crystal was washed with acetone and methanol, then reduced by etching with hydrogen peroxide followed by hydrogen fluoride. Typically, a H₂O₂ (35%) solution was used during 10 sec. at 20°C. After washing with H₂O, a solution of HF (10%) was used during 10 min at 20°C. The crystal was washed with dichloromethane and stored under CH₂Cl₂. This crystal, called Ge-H, was analyzed by X-ray photoelectron spectroscopy (XPS). The results are shown in Table 1.

### Step 2: surface grafting

The germanium crystal of step 1 was treated with a solution of alkene in mesitylene at reflux in a dry vessel and under a flow of argon. Typically, heptadecafluoro-1-decene (2%) was used, during 2h at 160°C. The crystal was rinsed with CH₂Cl₂ and n-pentane (soxhlet, 2h) and stored under CH₂Cl₂. This crystal called Ge-F₁₇, was analyzed by XPS. The results are shown in Table 1. A blank sample, called Ge-F_{17b}, was prepared by treatment with heptadecafluoro-1-decene (2%), but at 20°C (2h) and rinsing as above.

**Table 1: XPS analyses**

| | **% of elements** | | | | | |
|---|---|---|---|---|---|---|
| **Sample** | **O₁ₛ** | **C₁ₛ** | **S₂ₚ** | **F₁ₛ** | **Ge_{3d}** | **other** |
| **native Ge** | 47.29 | 26.04 | 7.88 | / | 16.89 | 1.91 (Na) |
| **Ge-H** | 11.00 | 38.82 | / | / | 50.18 | / |
| **Ge-F₁₇** | 31.30 | 40.35 | / | 8.04 | 20.31 | / |
| **Ge-F_{17b}** | 30.15 | 37.81 | / | 1.73 | 30.30 | / |

### Example 2: Surface grafting of an alkene on Ge-H crystal, followed by photoactivation and XPS control of reactivity.

### Step 1: surface activation

A germanium crystal was washed with acetone and methanol, then reduced by etching with hydrogen peroxide followed by hydrogen fluoride. Typically, a H₂O₂ (30%) solution was used during 10 sec. at 20°C. After washing with H₂O a solution of HF (10%) was used during 20 min at 20°C. The crystal was washed with dichloromethane and used directly for the next step.

### Step 2: surface grafting

The germanium crystal of step 1 was treated with pure octadecene at reflux during 2h. Then, the crystal was rinsed with hexane, ethanol and dichloromethane (2h using soxhlet). The crystal called Ge-A₄, was analyzed by XPS. The results are shown in Table 2.

### Step 3: surface photoactivation

A solution (1.5 mL) of 4-(p-azidophenyl)butanoic acid activated as succinimidyl ester in benzene/ether (1:1) (-3 mg/ml) was sprayed onto the germanium crystal of step 2. After air drying, half of the crystal (the "blank" Ge-A₄ab) was covered with a black cardboard. The other half (Ge-A₄a) was irradiated during 2h with 3 UV lamps (254 nm) placed at a distance of 7 cm. The crystal was then rinsed for 5 min in CHCl₃ and for 10 min in tetrahydrofuran, under smooth shaking. The two parts was analyzed by XPS. The results are shown in Table 2.

### Step 4: surface reaction with a fluorinated label

The germanium crystal of the step 3 was incubated, under shaking, with a solution (30mL) of 3, 5*-bis* (trifluoromethyl) benzyl amine (0.7% in CH₂Cl₂) during 3h at 50°C. The crystal was rinsed two times (10 min and 5 min) with CH₃CN under smooth shaking. The XPS analysis was realised on blank part (Ge-A₄a_{b}F₆) and also on the grafted part (Ge-A₄aF₆). The XPS results are shown in Table 2.

**Table 2: XPS analyses**

| | **% of elements** | | | | | |
|---|---|---|---|---|---|---|
| **Sample** | **O₁ₛ** | **C₁ₛ** | **N₁ₛ** | **F₁ₛ** | **Ge_{3d}** | **other** |
| **native Ge** | 40.10 | 28.16 | 1.67 | / | 29.79 | 0.26 (Si) |
| **Ge-A₄** | 31.41 | 43.07 | 0.63 | / | 24.89 | / |
| **Ge-A₄a** | 29.86 | 56.96 | 4.66 | / | 8.52 | / |
| **Ge-A₄a_{b}** | 33.68 | 42.51 | 1.41 | / | 22.40 | / |
| **Ge-A₄aF₆** | 28.51 | 51.99 | 5.06 | 2.35 | 12.09 | / |
| **Ge-A₄a_{b}F₆** | 21.08 | 58.25 | 2.59 | 0.33 | 17.75 | / |

### Example 3: Surface grafting of a functionalized alkene, followed by XPS control of reactivity.

### Step 1: surface activation

As described in Example 2.

### Step 2: surface grafting (thermal activation)

The germanium crystal of step 1 was treated with a functionalized alkene, called SD15, dissolved in mesitylene, at 160°C during 2h, under a flow of argon. Typically, a solution of 0.2 mmol in 5 ml was used. The crystal was rinsed with CH₂Cl₂ (15 x 1 mL, 3 min shaking) and stored under CH₂Cl₂. This crystal, called Ge-OTS, was analyzed by XPS. The XPS results are shown in Table 3.

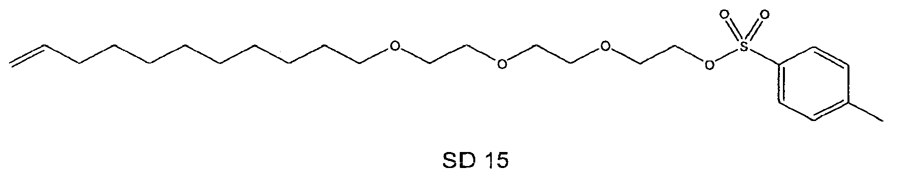

### Step 3: surface reaction with fluorinated label

The germanium crystal of step 2 was incubated, under shaking, with a solution of 3, 5-*bis* (trifluoromethyl) benzyl amine during 2h at 20°C. Typically, a 10⁻³ M solution in CH₃CN-PBS buffer (1:1) was used. The crystal was washed with water (10 x 1 mL, shaking 2 min) and stored under CH₂Cl₂. This sample was called Ge-F₆ (see XPS analysis in Table 3). The "blank" sample (Ge-F_{6b}) resulted from the incubation of Ge-H with the amine solution and rinsing as described above .The XPS results are shown in Table 3.

**Table 3: XPS analyses**

| | **% of elements** | | | | | |
|---|---|---|---|---|---|---|
| **Sample** | **O₁ₛ** | **C₁ₛ** | **S₂ₚ** | **F₁ₛ** | **Ge_{3d}** | **other** |
| **Ge-H** | 11.00 | 38.82 | / | / | 50.18 | / |
| **Ge-OTS** | 18.30 | 69.40 | 1.40 | / | 10.40 | / |
| **Ge-F₆** | 23.40 | 59.00 | / | 2.20 | 15.40 | / |
| **Ge-F_{6b}** | 29.60 | 38.50 | / | / | 31.90 | / |

### Example 4: Surface grafting of functionalized alkene, followed by LSC control of reactivity.

### Step 1: surface activation

As described in Example 2.

### Step 2: surface grafting (photochemical activation)

The germanium crystal of step 1 was treated with a functionalized alkene, called SD 15, dissolved in mesitylene, under irradiation at room temperature. Typically, a solution of 0.2 mmol of SD 15 in 5 mL of mesitylene was used. The crystal was immerged and irradiated with visible light (500 W) during 12 h. The crystal was then rinsed with CH₂Cl₂ (10 X 1 mL, 5 min shaking) and stored under CH₂Cl₂. This crystal was called Ge-OTS (hv). The blank sample was similary prepared, but with omitting SD 15.

### Step 3: surface reaction with tritiated label

The germanium crystal of step 2 was incubated, under shaking, with a solution of (L) - 4, 5 - [³H] - lysine (called Lys*), at room temperature. Typically, a 10⁻³ M solution of Lys* in phosphate buffer (pH = 8) was used. The crystal Ge-OTS (hv) wad immersed during 12 h, then washed with water several times. The radioactivity associated to sample was measured by liquid scintillation counting (LSC): Ge-OTS (hv) + Lys* = 434 pmol / cm² (blank sample = 150 pmol / cm²).

### Example 5: Drug binding on human serum albumin.

### Step 1: surface activation

As described in Example 2.

### Step 2: surface grafting

As described in Example 2.

### Step 3: surface photoactivation

As described in Example 2.

### Step 4: receptor binding

In this example, the modified germanium internal reflection element was sealed in a liquid flow cell; human serum albumin (1.5 mg/ml) was introduced in the cell and incubated for 30 minutes in the presence of the activated surface. After 30 minutes the excess was washed with the PBS buffer. Figure 1 shows the spectrum of the bound protein. Interestingly, the shape of the so-called amide I (1640 cm⁻¹) and amide II (1540 cm⁻¹) bands is characteristic of the protein.

### Step 5: ligand binding on the receptor

Sulfadimethoxine was used here as a model of drug binding onto human serum albumin. It was injected at a concentration of 1 mM into the flow cell and spectra were recorded as a function of the time. Every 40 second a new spectrum (256 scans, resolution 2 cm⁻¹) was stored. When binding on the receptor, the drug comes in close contact with the reflecting interface, i.e. within the range of the evanescent field, and the spectrum of the drug becomes visible. We report on Figure 2 the monitoring of the intensity variation at 1550 cm⁻¹ due to the strong absorbance of sulfadimethoxine at this wavenumber. Figure 2 demonstrates the reversible binding of sulfadimethoxine on the receptor. It also demonstrates that the receptor, because of the reversibility of the reaction, can be used several times. ln fact, experiments repeated in the course of two days demonstrated that the receptor is very stable.

### Example 6: biotin binding on avidin

### Step 1: surface activation

As described in Example 2.

### Step 2: surface grafting

As described in Example 2.

### Step 3: surface photoactivation

As described in Example 2.

### Step 4: receptor binding

In this example, the activated germanium internal reflection element was sealed in a liquid flow cell; avidin (1.5 mg/ml) was introduced in the cell and incubated for 30 minutes in the presence of the activated surface. After 30 minutes the excess was washed with the PBS buffer.

### Step 5: ligand binding on the receptor

Biotin binding on avidin was monitored by infrared spectroscopy. 150 spectra (resolution 2 cm⁻¹, 256 scans) were recorded as the concentration was decreased from 1 mM to 10⁻¹² M. Biotin binding was monitored at 1627 cm⁻¹. Figure 3 reports the absorbance as a function of the spectrum number and as a function of biotin concentration.

It can be observed in Figure 3 that a clear transition in the absorbance level can be observed for a concentration of 10⁻¹⁰ M in biotin.

## Claims

1. Device suitable for the investigation of ligand-receptor interactions, in particular for the investigation of an analyte target interaction such as biological and chemical molecules and organic components and their interaction with surfaces, consisting of an attenuated total internal reflection element, transparent in the infra-red and of which at least one surface is reduced and covalently grafted with an alkene able to immobilize the receptor, wherein said alkene is optionally substituted by one or more substituent selected from alkyl, haloalkyl, halo, alkenyl, cyano, epoxy, thio, amino, hydroxyl, isocyano, isothiocyano, carboxy, polyalkoxy, alkyarylsulphoxypolyalkoxy, or heteroaryloxycarbonylakylpolyalkoxy.

2. Device according to claim 1, wherein the optionally substituted alkene is of Formula 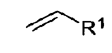 wherein R¹ is selected from CₙH₂ₙ₊₁, CₙF₂ₙ₊₁, -(CH₂)ₘ-(O-CH₂-CH₂)ₚ-OR², wherein R² is selected from C₁₋₃alkyl, alkyarylsulphoxide, heteroaryloxycarbonylakyl, 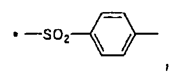 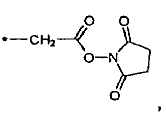 or mixture thereof,
wherein n is an integer from 3 to 50, wherein m is an integer from 0 to 20, and wherein p is an integer from 3 to 20.

3. Device according to claim 1, wherein the alkene is covalently coupled with a multifunctional spacer-arm of the general formula
Z¹-(CH₂)_{q}-Z² wherein q is 2 to 12; or
Z¹-CH₂-(O-CH₂-CH₂)_{q}-OCH₂-Z² wherein q' is 0 to 5;
wherein Z¹, Z² are each independently chosen from N₃-aryl-; diazirinyl; -CO₂H and N-hydroxysuccinimidyl ester thereof; -CH₂-NH₂ and N-maleimidyl derivative thereof; -CH₂OH and tosylates thereof; -CH₂-SH and dithiane derivatives thereof; -CH₂-N=C=O; -CH₂N=C=S;

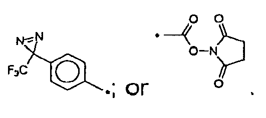

4. Device according to any of the claims 1 to 3, wherein the attenuated total internal reflection element is made from germanium.

5. Device according to any of the claims 1-4, wherein the attenuated total internal reflection element is a crystal, preferably having a trapezoidal, hemi - cylindrical, fiber or rod shaped geometry, or polyhedral form.

6. Use of a device according to any of the claims 1-5 for studying ligand-receptor interactions, in particular biological molecules or organic components or their interactions or complexations or reactions with biological molecules or organic components or water-soluble molecules at or in the grafted organic molecule.

7. Method for the construction of a device according to any of the claims 1-5 comprising the steps of:
- surface reduction of at least one surface of an attenuated total internal reflection element,
- surface grafting with an alkene of the reduced surface obtained in the previous step, and
- coupling a receptor via covalent fixation on the grafted attenuated total internal reflection element,
wherein said alkene is optionally substituted by one or more substituent selected from alkyl, haloalkyl, halo, alkenyl, cyano, epoxy, thio, amino, hydroxyl, isocyano, isothiocyano, carboxy, polyalkoxy, alkyarylsulphoxypolyalkoxy, heteroaryloxycarbonylakylpolyalkoxy.

8. Method according to claim 7. wherein the reduction step is performed chemically or electrochemically.

9. Method according to claim 7 or 8, wherein the surface grafting is performed through covalent coupling with an optionally substituted alkene is of Formula 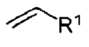 wherein R¹ is selected from CₙH₂ₙ₊₁, CₙF₂ₙ₊₁, -(CH2)ₘ-(O-CH₂-CH₂)ₚ-OR², wherein R² is selected from C₁₋₃alkyl, alkyarylsulphoxide, heteroaryloxycarbonylakyl, 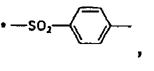 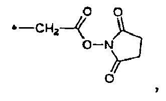 or mixture thereof,
wherein n is an integer from 3 to 50, wherein m is an integer from 0 to 20, and wherein p is an integer from 3 to 20.

10. Method according to claim 9, further comprising the covalent coupling on the alkene of a multifunctional spacer-arm of the general formula
Z¹-(CH₂)q-Z² wherein q is 2 to 12; or
Z¹-CH₂-(O-CH₂-CH₂)_{q}-OCH₂-Z² wherein q' is 0 to 5;
wherein Z¹, Z² are each independently chosen from N₃-aryl-; diazirinyl; -CO₂H and N-hydroxysuccinimidyl ester thereof; -CH₂-NH₂ and N-maleimidyl derivative thereof; -CH₂OH and tosylates thereof; -CH₂-SH and dithiane derivatives thereof; -CH₂-N=C=O; -CH₂N=C=S; 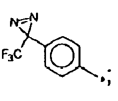 or

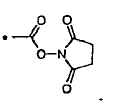

11. Method according to any of the claims 7-10, wherein a receptor is immobilized on the organic molecule.

12. Device obtainable by a method according to any of the claims 7-11.

13. Method for reducing a surface of an attenuated total internal reflection element, comprising the step of stripping the surface with an oxidizing agent and reducing the stripped surface with a reducing agent.

14. Method according to claim 13 for the reduction of an attenuated total internal reflection element, by the consecutive treatment of a surface thereof with H₂O₂ and HF.

15. Method according to claim 13 for the reduction of a germanium crystal by the consecutive treatment of a surface thereof with H₂O₂ and then HF.

16. Method for studying ligand-receptor interactions, in particular biological molecules or organic components or their interactions or complexations or reactions with biological molecules or organic components or water-soluble molecules at or in the grafted organic molecule, using a device according to claim 12, comprising the steps of
- installation of said device in a FTIR cell
- conducting a flux of potential ligands, preferably a water-containing solution, on said device surface
- analysis of the infra-red spectrum obtained after submitting a FTIR beam through said cell
- reuse of said device surface by application of a solution of free ligand.
